# EUROPEAN PATENT SPECIFICATION

(11) **EP 3 870 205 B1**
(45) Date of publication and mention of the grant of the patent: **31.07.2024**
(21) Application number: 19875861.7
(22) Date of filing: 23.10.2019
(51) Int. Cl.: A61K 38/10, C07K 7/00, C07K 7/08, C07K 14/16, C07K 16/10, C12Q 1/68, G01N 33/53, G01N 33/543, G01N 33/68

(54) **LATERAL FLOW ASSAYS FOR DIFFERENTIAL ISOTYPE DETECTION ASSOCIATED WITH ZIKA VIRUS**
LATERALE FLUSSTESTS ZUR DIFFERENZIELLEN ZIKA-VIRUS ASSOZIIERTEN ISOTYP-ERKENNUNG
TESTS DE FLUX LATÉRAL POUR LA DÉTECTION DIFFÉRENTIELLE DES ISOTYPES ASSOCIÉS AU VIRUS ZIKA

(30) Priority: 24.10.2018 US 201862749776 P
(43) Date of publication of application: 01.09.2021
(73) Proprietor: OraSure Technologies, Inc., Bethlehem, PA 18015-1360 (US)
(72) Inventor: REED, Michael, Bethlehem, PA 18015 (US); DAVIS, Manli, Bethlehem, PA 18015 (US); GUILLON, Geraldine, Bethlehem, PA 18015 (US); FISCHL, Mark, Key West, FL 33040 (US)
(74) Representative: Jacobi, Markus Alexander
(86) International application number: PCT/US2019/057568
(87) International publication number: WO 2020/086664

(56) References cited:
- WO-A1-2016/022071
- WO-A1-2019/032669
- WO-A2-01/42788
- CN-A- 103 267 844
- CN-A- 103 983 776
- CN-A- 105 954 512
- CN-A- 108 445 210
- US-A1- 2009 024 060
- US-A1- 2013 309 656
- US-A1- 2018 238 881
- US-B2- 8 062 908
- US-B2- 9 068 981
- JIHOO LEE ET AL: "Enhanced performance of an innovative dengue IgG/IgM rapid diagnostic test using an anti-dengue EDI monoclonal antibody and dengue virus antigen", SCIENTIFIC REPORTS, vol. 5, no. 1, 1 December 2015 (2015-12-01), XP055636937, DOI: 10.1038/srep18077
- YEONG HOON KIM ET AL: "Development of a Rapid Diagnostic Test Kit to Detect IgG/IgM Antibody against Zika Virus Using Monoclonal Antibodies to the Envelope and Non-structural Protein 1 of the Virus", KOREAN JOURNAL OF PARASITOLOGY., vol. 56, no. 1, 28 February 2018 (2018-02-28), KR, pages 61 - 70, XP055636952, ISSN: 0023-4001, DOI: 10.3347/kjp.2018.56.1.61

## Description

### Cross Reference to Related Applications

This application claims priority to U.S. Application No. 62/749,776, filed on October 24, 2018.

### Field of the Invention

This invention relates to diagnostic methods and devices for detection of antibody classes associated with acute immune responses by lateral flow techniques as defined by the claims.

### Background

Acute immune responses may be indicative of many conditions ranging from infectious disease to cancer to tissue injury. When these responses are being determined by differential immunoglobulin Fc class or isotype detection (e.g., IgG vs. IgM classes), it is critical that interactions of one isotype with assay components does not interfere with the interactions of another isotype with assay components. This is a particular risk if one isotype is in excess of the other and/or has a higher affinity for assay components or when reagents cannot distinguish the reactive antibody isotypes. For example, when testing for an immune response to a virus, such as Zika, it is desirable to detect IgM antibodies associated with the acute, recent exposure or infection, and not the presence of the IgG antibodies of a matured immune response.

Immunoassays are used to quantify molecules of biological interest based on specificity and selectivity of antibody reagents or targets. For point-of-care testing, immunoassays are often used in a lateral flow assay format. *See, e.g.,* U.S. Patent No. 8,062,908, U.S. Patent No. 7,541,194, U.S. Patent No. 7,192,555 and U.S. Patent No. 6,303,081. However, there are few diagnostic tools to identify acute infections.

IgM antibodies, are often indicative of an acute immune response to an infection. Because IgM antibodies are typically the first isotypes of antibodies produced by the immune system in response to an initial exposure to an antigen, immunoassays that detect antigen-specific IgM are useful for identifying acute infections because they detect antibodies produced during the first few days after a primary infection and persist for a few months. As the immune response to a particular antigen matures, a switch to other isotypes that have structurally different Fc-regions (e.g. IgA, IgE, IgG1, IgG2, IgG3, IgG4) occurs producing mixed class responses. In some infections, the host B cells may even proceed to completely stop producing the IgM isotype. The sequences and folding of the N-terminal end of the antibody determines the binding specificity, is formed from multiple genetic cassettes and fine-tuned by somatic mutations acquired in an AID-dependent process during the immune response. During this process some B cells also use the same AID enzyme to loop out intervening DNA downstream (3') of end of the Variable region which contains the IgM Fc constant region in a single gene (exon), excise it and relegate to a "downstream isotype (*e*.*g*. IgG1, etc.). This is a permanent change at the DNA level in a given B cell and it cannot again make IgM. Thus, additional time in a sustained infection allows a maturation of response, including a higher concentration of specific antibodies and a higher population of higher affinity antibodies of isotypes other than IgM.

To detect a particular class of antibodies from a given sample, such as IgM, it is sometimes necessary to remove interference caused by the presence of other higher abundance or higher affinity antibody classes, such as IgG. These Fc antibody classes or isotypes as they are called to those familiar in the art, will preferentially bind assay components and prevent detection of antibodies of interest, such as IgM. Therefore, there is a need for an assay test design that eliminates interference from a class or classes of antibodies to allow for the detection of another class of antibodies.

In some cases, lateral flow assays may also suffer from issues related to cross-reacting antibodies that can lead to false positive results. For example, Zika virus and Dengue virus are closely related flaviviruses. The non-structure protein 1 (NS1) of Zika and Dengue viruses exhibit high homology. Antibodies against the NS1 protein of Dengue may also recognize NS1 from Zika, and vice versa. Therefore, there is a need to also differentially detect antibody isotypes to a target of interest within a sample apart from cross-reacting antibodies. CN-A 105954512 describes a detection strip or kit for Zika virus detection by means of the fast immunochromatography method. US-A 2018/0238881 describes a further immunochromatography lateral flow assay for identifying Zika virus detection and straifying it into early and late Zika virus infection.

### Summary of the Invention

The invention relates to lateral flow assay test strips as defined by the claims. Furthermore assay methods, in particular, lateral flow assay methods for detection of an antibody isotype associated with acute infection, and to lateral flow assay strips for use in the methods are described herein, but not claimed as such.

Lateral flow assay test strips according to the invention are designed to differentiate between IgG and IgM isotypes in a liquid biological sample which bind to Zika NS1 polypeptides. Lateral flow assay test strips according to the invention are defined by the claims.

Lateral flow assay strips of the invention are also configured to reduce cross-reactive antibodies from binding to the labeled antigen. Such lateral flow assay strips contain a blocker area comprising Dengue NS1 polypeptides to bind antibodies that cross-react with the labeled antigen (i.e. labeled Zika NS1 polypeptides).

The invention also relates to lateral flow assay devices. Lateral flow assay devices according to the invention comprise an assay portion housing a lateral flow strip according to the invention and an opening to view the test zone and optional control zone. The invention also relates to lateral flow assay kits including lateral flow assay strips of the invention and a developer solution. For the avoidance of doubt, embodiments relating to other viruses (besides Zika), antigens (besides Zika NS1 polypeptides), antibody isotypes (besides IgG and IgM), blocker areas (besides those comprising Dengue NS1 polypeptides) etc are described in the "Detailed Description" but are not claimed as such.

### Brief Description of the Drawing

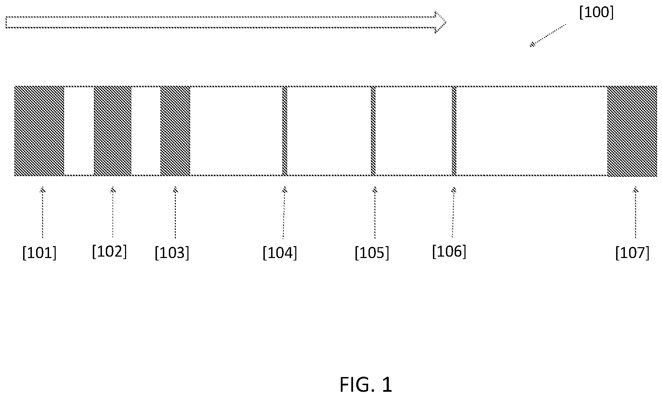

FIG. 1 shows a representative schematic of a lateral flow assay test strip [100] according to the invention. After application of the sample at the sample receiving area [101], the sample moves across the test strip [100] in the direction indicated by the arrow, flowing across a blocker area [102], a conjugate area [103] where antibodies in the biological sample bind to a labeled antigen, and subsequently flows through to a first capture zone [104] capturing labeled complexes by antibody isotype, followed by a second capture zone [105] capturing labeled complexes with acute infection antibody isotypes, followed by an control zone [106] to collect any uncaptured labeled antigen, and finally reaches an absorption pad [107].

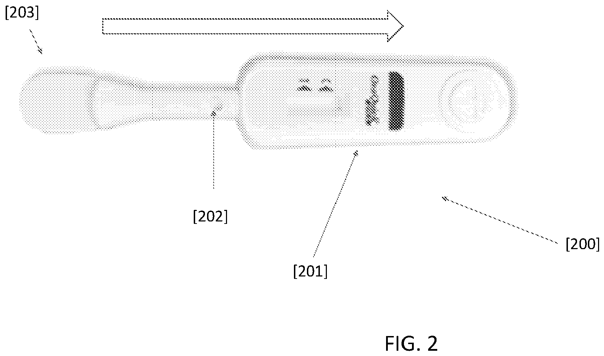

FIG. 2 illustrates a lateral flow device for the detection of an infection, for example a Zika infection, according to the invention. The lateral flow device [200] has a housing [201] with a sample port [202]. As shown, the lateral flow test device [200] may also contain an additional sample application member depicted here as a flat pad [203], which may act as a wick to deliver a liquid biological to a sample receiving area. The device [200] contains a lateral flow assay test strip [100] from FIG. 1, which is in flow communication with the flat pad [203]. As the sample moves up the lateral flow assay strip, the presence of labeled complexes may be visualized at the IgM capture line (labeled T on the housing [201]). Completion of the assay would be indicated at the control line (labeled C on the housing [201]). The direction of flow is indicated by the arrow.

### Detailed Description

Described are further lateral flow assays for detection of an antibody isotype associated with acute infection. A lateral flow assay method may comprise: a) contacts a liquid biological sample with a labeled antigen to form complexes with antigen-specific antibodies; b) captures labeled antigen-antibody complexes from step a) with an immobilized capture reagent which specifically binds to an antibody isotype associated with a mature immune response at a first capture zone; c) captures labeled antigen-antibody complexes from step a) with an immobilized capture reagent specific for an antibody isotype associated with an acute immune response at a second capture zone, downstream of the first capture zone; d) detects the captured complex from step c).

Methods, assay strips and devices as defined herein are particularly useful for early detection of infection where early treatment is needed. An assay method as described herein can identify early infection and differentiate from past infection of a disease by differentiating between at least two different antibodies, which bind to a particular disease antigen, according to isotype. For some assay methods as described herein, it is advantageous to first deplete the biological sample of isotypes associated with a mature response to detect the isotype associated with an acute response.

Diseases where the acute phase provides an opportunity for intervention to improve patient or population safety or in sample types where depletion of a class of antibody may boost sensitivity in certain assay formats are of particular interest. Such diseases include, for example, Arbovirus neurologic disease (e.g., WNV, EEE virus, and SLE virus infections), Arbovirus rash illness (e.g., dengue virus, CHIK virus, and Zika virus infections), CMV and EBV infectious mononucleosis, Hantavirus pulmonary syndrome, hepatitis A, B, and E virus infections, HIV-1 and HIV-2 infections, measles, rubella, mumps, Parvovirus B19 Fifth disease, Helicobacter pylori infection, Borrelia burgdorferi infection, HCV, and other viral infections. *See e.g.* Landry, Clin. and Vacc. Immun. Vol. 23, pp. 540-45, 2016. For example, the presence of IgM class anti-measles antibodies indicates an acute measles infection whereas the presence of IgG class anti-measles antibodies indicates previous exposure and immunity to measles. The presence of IgM antibodies is also an indicator of acute Hepatitis A virus (HAV) infection. HAV specific IgM antibodies are detectable in serum between 4 weeks to 6 months post infection. While the examples below are described with regard to the Zika virus, which is accoring to the presnet invention, an assay method may be used when differentiation between at least two different antibodies according to isotype which bind to a particular disease antigen assists or is needed for identification, determination and/or treatment of any infection.

An antibody, as is known in the art, refers to a heterodimeric glycoprotein polypeptide or complex of polypeptides, substantially encoded by an immunoglobulin gene or immunoglobulin genes, or fragments thereof. The recognized immunoglobulin genes include the kappa, lambda, alpha, gamma, delta, epsilon, and mu constant regions, as well as myriad immunoglobulin variable region genes. Light chains are classified as either kappa or lambda. Heavy chains are classified as gamma, mu, alpha, delta, or epsilon, which in turn define the immunoglobulin classes (isotypes), IgG, IgM, IgA, IgD, and IgE, respectively. The constant domains of the heavy chains make up the Fc region of an antibody. The Fc portion of the antibody determines the antibody class. Two different antibody isotypes, for example, IgM and IgG, may share similarity in the N terminal Variable region of the antibody which allow them to specifically bind to the same antigen, but differ in the downstream constant regions which have different effector functions and sequences.

Typically, an antibody is an immunoglobulin having an area on its surface or in a cavity that specifically binds to and is thereby defined as complementary with a particular spatial and polar organization of another molecule. The antibody can be polyclonal or monoclonal. Antibodies may include a complete immunoglobulin or fragments thereof. Fragments thereof may include Fab, Fv and F(ab')2, Fab', and the like. Antibodies may also include chimeric antibodies or fragment thereof made by recombinant methods.

An antigen is any compound capable of specifically binding to an antibody of interest. Specific binding between the antigen and the antibody means that the two molecules are related such that their binding with each other is capable of discrimination against binding between the antigen and non-specific antibodies, or binding between the antibody and a different antigen. The binding may be through chemical or physical means. A molecule may specifically bind to an aggregation of molecules; for example, an antibody raised against an immune complex of a second antibody and its corresponding antigen may be considered to be a specific binding partner for the immune complex.

Assay methods may be performed on a lateral flow assay strip. The methods further comprise contacting a lateral flow assay strip with a developer solution. Upon contact, the developer solution moves across the lateral flow assay strip. In a method, the liquid biological sample is placed in a developer solution, and the resulting developer solution comprising the biological sample moves across the assay strip to points downstream on the lateral flow assay strip. In another method as described herein, the liquid biological sample is placed on a sample receiving area of the lateral flow assay strip, and a developer solution contacts the lateral flow assay strip to facilitate the flow of the biological sample across the assay strip from the sample receiving area to points downstream.

The liquid biological sample taken for analysis using an assay method as described herein may be any liquid biological sample, such as a biological fluid, which may contain antibodies of interest. Examples of biological fluids include, but are not limited to, urine, blood, plasma, serum, oral fluids, sweat, semen, stool, sputum, cerebral spinal fluid, tears, mucus, amniotic fluid, breast milk and the like. Oral fluid is the liquid present in the oral cavity. Oral fluid is a mixture of saliva and oral mucosal transudate. Saliva is produced by the salivary glands. Oral mucosal transudate enters the mouth by crossing the buccal mucosa from the capillaries. Oral fluids contain both pathogens and antibodies. Biological fluid samples such as oral fluid, whole blood, blood plasma, and blood serum are preferred types of samples useable in assays of the invention. Each of these may be acquired using means and techniques known in the art.

Viral infection, such as infection from the Zika virus (according to the invention), can be detected in multiple matrices which may include liquid biological samples such as whole blood (venous or fingerstick), serum and plasma, oral fluid (e.g., saliva and oral mucosal transudate), urine, seminal fluid, and breast milk from patients with clinical signs and symptoms and/or epidemiological risk factors. A method for rapid collection and assay of oral fluids is disclosed in U.S. Patent No. 8,062,908. In a method as described hereindirected to measuring whole blood, an appropriate volume of blood is placed onto the device by a healthcare worker. The amount of liquid biological sample to be used can vary based on the liquid biological sample and the assay format.

The step of contacting the liquid biological sample with the labeled antigen occurs in a liquid phase. According to one method, the liquid biological sample and the labeled antigen are contacted in a solution on a conjugate pad or conjugate area on a lateral flow assay test strip. In a method, a developer solution, described below, is used and facilitates migration of the sample up the assay strip. The liquid biological sample is placed in contact with the labeled antigen in solution on the assay strip at the conjugate area. In methodsas described herein, the conjugate area is incubated with the labeled antigen and dried, and when the developer solution reaches the conjugate area, the labeled antigen is eluted off the matrix of the conjugate area, and available for binding to antigen-specific antibodies present in the biological sample. According to another method, the liquid biological sample and labeled antigen are contacted in solution prior to placement on a lateral flow assay test strip.

Antibodies present in the biological sample may be detected using labeled antigens. When a labeled antigen is used, a labeled complex, is formed by virtue of the antibody binding to the labeled antigen. The binding reaction to form a labeled antibody employs a specific binding antibody-antigen interaction, such as described above. The presence of the antibody at a downstream capture zone can be detected by detecting the presence of the label. through visual or instrumental detection.

The label may be any substance which is visible itself or capable of producing a signal that is detectable by visual or instrumental means. The selection of a particular label is not critical to the invention, but the label should be capable of generating a detectable signal either by itself, or be instrumentally detectable, or be detectable in conjunction with one or more additional signal producing components, such as an enzyme/substrate signal producing system.

As is known in the art the choice of the type of label involves consideration of the analyte to be detected and the desired means of detection. Detection of the detectable label will depend on the chosen moiety or reagent, and can be done by any of the methods known in the state of the art, for example, visual inspection, ultraviolet and visible spectrophotometry, fluorimetry, radioactivity counting or the like. In a preferred embodiment, when gold particles are used as detectable labels, the results of the assay are obtained and analyzed by visual inspection. Various labels known in the art and suitable for use in the methods as described hereininclude labels which are visible themselves or which produce signals through either chemical or physical means. Such labels can include enzymes and substrates, chromogens, catalysts, fluorescent compounds, chemiluminescent compounds, molecular beacons, carbon black, polystyrene beads, and radioactive labels. Other suitable labels known in the art include particulate labels such as colloidal metallic particles such as colloidal gold, colloidal non-metallic particles such as selenium or tellurium, dyed or colored particles such as a dyed plastic or a stained microorganism, organic polymer latex particles and liposomes, colored beads, polymer microcapsules, sacs, erythrocytes, erythrocyte ghosts, or other vesicles containing directly visible substances, and the like. U.S. Pat. No. 4,313,734 describes the use of gold Sols as labels for antibodies. Typically, a visually detectable label is used. This allows for direct visual or instrumental reading of the presence or amount of an analyte in the sample without the need for additional signal producing components at the test zone. Methods to attach labels to antigens such as polypeptides are known in the art. See e.g., Bioconjugate Techniques, Hermanson, Third Ed., 2013.

In a preferred assay method, the detectable label is a metal colloidal particle, such as gold microparticles or gold nanoparticles, which can be seen by the human eye as well as instrument read. In an assay method, colloidal particles used as a detectable label have a particle size diameter of between 20 nm and 80 nm. For an instrument read assay, the concentration of the particles is measured by measuring Optical Density (OD) at a specific wavelength using a spectrophotometer. OD is then used as a measure of the total amount of labeled antigen. In an assay method, the amount of labeled antigen should preferably be in excess of the antibody to be detected to allow for complete labeling.

In an assay method, the labeled antigen may be any labeled viral antigen. In methods as described herein, the labeled viral antigen recognizes antibodies of at least two different isotypes, that is, an isotype associated with an acute immune response, and an isotype associated with an acute immune response. In a lateral flow assay method, the viral antigen is a Zika virus antigen. According to the invention, the Zika virus antigen is a NS1 polypeptide or a fragment or variant thereof which can bind to IgM and IgG antibodies. In an assay according to the invention, the labeled antigen is a Zika NS1 gold conjugate formed by conjugating the Zika NS1 polypeptide with colloidal gold. In such a lateral flow assay method, the antibodies of the isotype associated with the mature immune response are Anti-Zika-NS1 IgGs, and the antibodies associated with the acute immune response are Anti-Zika-NS1 IgMs.

Capture reagents are reagents which specifically bind to antibodies of a specific class but not to antibodies of another class. The constant domains of the heavy chains make up the Fc region of an antibody. The Fc portion of the antibody determines the antibody class. Therefore, capture reagents for a particular isotype specifically recognize the Fc regions. The capture reagent may be an antibody or protein, or aptamer, or other specific affinity reagent that recognizes an isotype or class of antibodies. For example, Protein G, as known in the art, binds to all subclasses of human IgG, but not human IgM. Protein A, binds to only certain subclasses of IgG as well as other classes of antibodies. Antibodies specific for a class of antibody may also be used as capture reagents. For example, Anti-IgM antibody, or fragments thereof, may be used to capture IgM.

Capture reagents may be immobilized at a specified region on a surface to form a capture zone for a specific class of antibodies. For example, capture reagents may be immobilized on specific regions, areas, zones, or lines on a lateral flow assay test strip. Capture reagents may be immobilized onto capture zones of a lateral flow assay strip using techniques known in the art.

In an assay according to the invention, the antibody isotype associated with a mature immune response is IgG and the antibody isotype associated with an acute immune response is IgM. Both antibody isotypes bind to the same labeled antigen. The antibody isotypes may have different affinities for the antigen, and may be expressed at different levels in the liquid biological sample. In lateral flow assay according to the invention, the step of capturing the antibody isotype associated with a mature immune response occurs prior to the step capturing the antibody isotype associated with an acute immune response. Accordingly, in methods of the invention, the lateral flow assay strips used are configured so that the mature immune response antibody isotype capture zone is upstream of the acute immune response antibody isotype capture zone

An assay according to the invention is performed for the detection of Zika IgM antibody from a biological sample. In such a method, IgGs are captured from the biological sample first. Protein G is immobilized on the nitrocellulose membrane to capture human IgG upstream of the IgM capture line. Protein G exhibits high affinity to human IgG sub-classes 1-4 but not human IgM. In addition, the protein G line enables detection of anti-Zika NS1 IgG antibodies, and reduces the amount of IgG present in the sample as it flows through the device, and prior to sample crossing the IgM detection line. This IgG capture line is required for IgM sensitivity of the device.

A preferred lateral flow assay according to the invention for detection of anti-Zika IgM comprises the steps of:
a) contacting a liquid biological sample with a Zika NS1 gold conjugate to form labeled IgG and IgM antibody complexes;
b) reacting the labeled IgG antibodies with Protein G to capture labeled IgG antibodies,
c) reacting the labeled IgM with Anti-IgM antibody to capture labeled IgM downstream of the IgG capture zone, and
d) detecting the captured IgM.

In one assay according to the invention, a liquid biological sample flows through a lateral flow assay test strip of the invention, anti-Zika IgG and IgM antibodies from the liquid biological sample contact the colorimetric Zika NS1 gold conjugate from the conjugate pad; if the liquid biological sample contains anti-Zika NS1 IgG antibodies, the labeled complexes and unlabeled anti-Zika NS1 IgG antibodies will bind to Protein G at the first capture zone [104] resulting in a reddish-purple line at the first capture zine; if the liquid biological sample does not contain anti-Zika IgG antibodies no color is observed. Similarly, if the liquid biological sample contains anti-Zika NS1 IgM antibodies, the labeled complexes and unlabeled anti-Zika NS1 IgM antibodies will bind to anti-IgM antibody at the second capture zone [105], (or the Test line (T), as shown in FIG. 2) resulting in a reddish-purple line; if the liquid biological sample does not contain anti-Zika IgM antibodies, no color is observed. Zika NS1 gold conjugate that is not bound to either capture zone travels up the nitrocellulose to the control zone control (or the Control line (C), as shown in FIG. 2).

Some methods are further directed to reducing false-positives due to cross-reactive antibodies. Such methods include an additional step of contacting the liquid biological sample with at least one other antigen to bind cross-reactive antibodies prior to contacting the liquid biological sample with the labeled antigen. Cross-reactivity between antigens occurs when an antibody directed against one specific antigen binds with another, different antigen. The two antigens have similar epitopes, which allow the antibody for one antigen to recognize the other antigen. Cross-reactive antibodies are antibodies other than the antibodies of the infection being investigated that cross-react with antigen of the infection being investigated. In one method of the invention, the at least other antigen is present on the blocker area of the lateral flow strip. In another method of the invention, the at least other antigen is added to the developer solution. In other methods of the invention, the at least one other antigen is contacted with the biological sample simultaneously with the labeled antigen or is added after the biological sample has been contacted with the labeled antigen, but prior to the steps of capturing labeled antigen-antibody complexes.

In a method, the labeled antigen is a labeled Zika NS1 polypeptide and the at least one other antigen is a Dengue NS1 polypeptide. A method for detection of anti-Zika IgM, a method comprises the steps of:
a) contacting a liquid biological sample with a Dengue NS1 polypeptide
b) contacting a liquid biological sample with a Zika NS1 gold conjugate to form labeled IgG and IgM antibody complexes;
c) reacting the labeled IgG antibodies with Protein G to capture labeled IgG antibodies,
d) reacting the labeled IgM with Anti-IgM antibody to capture labeled IgM downstream of the IgG capture zone, and
e) detecting the captured IgM.

In one embodiment of the above method, a Dengue NS1 polypeptide is added to the developer solution. In such a method, when the liquid biological sample is added to the developer solution, the sample comes into contact with a Dengue NS1 polypeptide in the solution prior to flowing onto a lateral flow assay strip. In another such method, when the liquid biological sample is added to the sample port, the sample comes into contact with a Dengue NS1 polypeptide in the solution on the lateral flow assay strip at the sample port. In another embodiment of the above method, a Dengue NS1 polypeptide is present at the blocker area, and when the biological sample, along with the developer solution migrates to the blocker area, a Dengue NS1 polypeptide is eluted off of the matrix of the blocker area and contacts the biological sample in solution. Without wishing to be bound by any particular theory, it is believed that pre-binding to Dengue NS1, blocks cross-reactive anti-Dengue NS1 antibodies from later binding to the Zika NS1.

A lateral flow assay test strip is to detect acute infection. Lateral flow chromatography assays, strips and devices are well known to those of skill in the art (see, *e.g.,* U.S. Pat. Nos. 5,569,608, 5,120,643, 5,656,503, 4,855,240, and 5,591,645, British Patent GB 2204398A, and European patent EP 0323605 B1) and such assays are commercially available on a retail or OEM basis for numerous analytes.

As shown in FIG. 1, a lateral flow assay test strip [100] according to the invention comprises: a)a sample receiving area [101] for a biological sample, b) a blocker area [102] downstream of the sample receiving area, c) a conjugate area [103] on the lateral flow assay test strip downstream from the blocker area, containing a labeled antigen which specifically binds to antibodies in the biological sample, d) a first capture zone or capture line [104] on the lateral flow assay test strip downstream from the conjugate area contains a capture reagent to specifically capture an antibody isotype associated with a mature immune response, e) a second capture zone or capture line on the lateral flow assay test strip downstream from the conjugate area contains a capture reagent to specifically capture an antibody isotype associated with an acute immune response, f) optionally, a control zone or control line [106] on the lateral flow assay test strip downstream from the second capture zone to indicate assay completion, and g) optionally, an absorbent pad [107] in flow communication with the lateral flow assay test strip and located downstream from the second capture zone and optional control zone.

A lateral flow assay test strip refers to a test strip utilized for lateral flow chromatography. Lateral flow (chromatography) assays typically involve the application of a liquid biological sample suspected of containing an analyte to be detected to a sample receiving area of a lateral flow (immunochromatographic) assay strip. The assay strip comprises a matrix material (e.g., paper, nitrocellulose, etc., see, e.g., U.S. Pat. No. 5,569,608) through which the test fluid and analyte suspended or dissolved therein can flow by capillary action from the sample receiving area to one or more capture zones where a visible signal, or absence of such, reveals the presence or absence of the analyte. Typically, the assay strip will include means for specifically binding the analyte to be detected with its specific binding partner (e.g., where the analyte is an antibody, the binding partner is an antigen,) which is modified with a detectable label. If analyte is present in the liquid biological sample, it will combine with its labeled binding partner to form a complex which will flow along the strip to a capture zone, where the labeled antibody will be detected.

A sample receiving area of the lateral flow assay test strip refers to the area of the lateral flow assay test strip to which a sample is first applied. In addition to receiving the sample the functions of the sample receiving area may include, for example: pH control/modification and/or specific gravity control/modification of the sample applied, removal or alteration of components of the sample which may interfere or cause non-specific binding in the assay, or to direct and control sample flow to the test region. For example, a collection pad of a device may be used to collect an oral fluid sample from a subject's mouth, and then is placed in a vial of developer solution. The collection pad wicks up the developer solution carrying along with it the oral fluid sample for the lateral flow assay test. For whole blood, serum or plasma collection, the sample is pipetted directly onto the sample receiving area or collection, and the collection pad is subsequently placed in the developer vial to flow through the lateral flow assay test and thereby transport the sample. In a particular assay according to the invention, whole blood is directly applied to the sample application area, for example, a drop of whole blood (5 µL to 50 µL) obtained from a fingerstick or venipuncture or needle without any type of prior dilution or treatment.

The sample receiving area may be a sample pad and may be made from any material capable of receiving the liquid biological sample and absorbing the liquid sample when applied and of passing the liquid sample to the blocker pad. Sample pad materials suitable for use in assay strips of the invention also include those application pad materials disclosed in U.S. Pat. No. 5,075,078. Suitable materials for the sample application area include, but are not limited to, hydrophilic polyethylene materials or pads, acrylic fiber, glass fiber, filter paper or pads, desiccated paper, paper pulp, fabric, and the like.

Alternatively, the sample receiving area may be the flat pad [203], as shown in FIG. 2. The sample receiving area may be comprised of an additional sample application member (e.g., a wick). Thus, in one aspect, the sample receiving zone can comprise a sample application pad as well as a sample application member. Often the sample application member is comprised of a material that readily absorbs any of a variety of fluid samples contemplated herein, and remains robust in physical form. Frequently, the sample application member is comprised of a material such as white bonded polyester fiber. Moreover, the sample application member, if present, is positioned in fluid-flow contact with a sample application pad. This fluid flow contact can comprise an overlapping, abutting or interlaced type of contact. The sample application member may also be treated with a hydrophilic finishing agent. Often the sample application member, if present, may contain similar reagents and be comprised of similar materials to those utilized in exemplary sample application pads. The liquid biological sample may be applied to the flat pad [203] directly. For example, the flat pad [203] may be inserted in the mouth of a subject, and be used as a collection pad for oral fluids. Other fluids, for example blood or serum may be directly placed on the flat pad [203]. The liquid biological sample may also be diluted in a developer solution in a vial, and the flat pad [203] may be placed in the vial.

The assay strips of this invention can optionally include a blocker pad. As known in the art a blocker pad contains reagents to ensure minimal reactivity within the device to nonspecific or interfering substances that are present in various sample matrices. A blocker pad can be composed of a wide variety of materials as long as they do not impede flow of oral fluid downstream to the lateral flow assay test strip. Such materials include, but are not limited to, paper, cellulose, nitrocellulose, polyester, glass fiber, and the like. Materials may be selected to reduce or eliminate backflow of reagents or oral fluid from the chromatographic test strip to the capillary matrix. With both buffering reagents and salts present the formulation contained in a blocker pad helps adjust the pH and ionic strength of the final reaction at the test line. A blocker pad can also be impregnated with buffers to adjust the sample pH of the liquid biological sample as it flows and for compatibility with the lateral flow assay.

A blocker pad can also include one or more blocking reagents that reduce cross-reactivity and thereby reduce the occurrence of false positives. In one lateral flow assay strip of the invention, the blocker area comprises at least one other antigen to bind cross-reactive antibodies. iln one lateral flow assay strip of the invention for use in detecting anti-Zika antibodies, labeled antigen is a labeled Zika NS1 polypeptide and the at least one other antigen is a Dengue NS1 polypeptide.

Other exemplary blocking reagents on the blocker pad include, but are not limited to, bovine serum albumin (BSA), methylated BSA, casein, nonfat dry milk, deoxycholate, and n-lauroyl sarcosine. The blocking solution may also contain surfactants, preservatives and other reagents to enhance flow across the test strip, improve assay results and protect sample integrity. A blocker pad is made by applying an appropriate volume of blocking solution onto the pad and drying before positioning it on the lateral flow assay test strip.

The conjugate area or conjugate pad of a lateral flow assay test strip serves to maintain label reagents and control reagents in a stable state and to facilitate their rapid and effective solubilization, mobilization and specific reaction with analytes of interest potentially present in the liquid biological sample. In a lateral flow assay test strip according to the invention, the conjugate area can be a conjugate pad. A conjugate pad is positioned on a lateral flow assay test strip such that the liquid biological sample must pass across or though the conjugate pad in order to migrate to the test zones or lines. Alternatively, a conjugate area can be woven into the lateral flow assay test strip or can be placed in-line, in the same place, as the lateral flow assay test strip. As with a blocker pad, a conjugate pad can be fashioned out of any convenient material (e.g., nitrocellulose) that is compatible with the assay and that does not substantially impede flow of the oral fluid and reagents. Other conjugate pad materials suitable for use by the present invention include those chromatographic materials disclosed in U.S. Pat. No. 5,075,078.

In a lateral flow assay test strip according to the invention, the conjugate pad carries the specific antigen conjugated to a label as well as release and stabilization agents to allow for detection of antigen at a test zone or line. The conjugate pad also provides some level of control through suppressing non-specific binding events within the assay device. In a lateral flow assay test strip according to the invention, the conjugate pad is treated with antigen-label solution that includes blocking agents and label-stabilizing agents. Blocking agents include those discussed above. Stabilizing agents are readily available and well known in the art, and may be used, for example, to stabilize labeled reagents.

Assay strips according to the invention comprise a matrix material (e.g., paper, nitrocellulose, etc., see, e.g., U.S. Pat. No. 5,569,608) across which the test fluid and analyte suspended or dissolved therein can flow by capillary action. Matrix materials can include, but are not limited to, natural, synthetic, or naturally occurring materials that are synthetically modified, such as polysaccharides (for example, cellulose materials such as paper and cellulose derivatives as cellulose acetate and nitrocellulose); polyether sulfone; nylon; silica; inorganic materials, such as deactivated alumina, diatomaceous earth, MgS0₄, or other inorganic finely divided material uniformly dispersed in a porous polymer matrix such as vinyl chloride, vinyl chloride- propylene copolymer, and vinyl chloride-vinyl acetate copolymer; cloth, both naturally occurring (for example, cotton) and synthetic (for example, rayon); porous gels, such as silica gel, agarose, dextran, and gelatin; polymeric films, such as polyacrylamide, and the like. In a preferred assay strip of the invention, the matrix material is a nitrocellulose membrane. Different types of commercially available nitrocellulose membranes (e.g., Millipore, HF90, GE FF120, Sartorius) possess different absorbent capacity and capillary flow.

In a lateral flow assay test strip, the matrix material may be adhered to a laminate backing. The backing may be a plastic material, such as e.g. Mylar or PVC or polystryrene. In preparing assay strips, the matrix material may be laminated onto a plastic card using techniques known in the art. Nitrocellulose membranes with laminate backings are commercially available.

Assay strips according to the invention have two or more capture zones. In lateral flow assay strips of the invention, capture zones contain immobilized capture reagent to specifically capture antibodies by isotype. Techniques for protein immobilization to create capture zones onto lateral flow matrices are known in the art. In a lateral flow assay test strip according to the invention, a first capture zone on the lateral flow assay test strip is downstream from the conjugate area to specifically capture antibodies of the first isotype. The first capture zone is upstream of the second capture zone. The capture zones and any control zones present should be separated sufficiently enough (e.g. about 5mm) from one another to visually distinguish the different zones.

In a lateral flow assay test strip according to the invention the antibodies associated with a mature immune response may be IgG. The capture zone for IgG, also known as the IgG test line, will bind IgG present in the sample tested. Protein G is binds to all the subclasses of human IgG. In a lateral flow assay test strip according to the invention, the first capture zone comprises the zone on the nitrocellulose membrane striped with Protein G. In such a lateral flow assay test strip, the first capture zone serves to substantially deplete IgG antibodies from the biological sample. Additional capture zones may be included for further isotype detection and/or differentiation.

In a lateral flow assay test strip according to the invention, a second capture zone on the lateral flow assay test strip is placed downstream from the first capture zone. In a lateral flow assay test strip according to the invention the antibody isotypes associated with an acute immune response may be IgM. The capture zone for IgM, also known as the IgM test line, will bind IgM present in the sample tested. In a lateral flow assay test strip according to the invention, the second capture zone comprises the zone on the nitrocellulose membrane striped with an anti-IgM antibody.

The optional control zone, or Control Line (C) contains antibodies specific for the labeled antigen, which captures the excess antigen label, demonstrating that the fluid has migrated adequately through the device. A reddish-purple line will appear at the C Line during the performance of all valid tests whether or not the sample is reactive or non- reactive for infection. In a lateral flow assay test strip according to the invention for the detection of anti-Zika IgM, the control zone may comprise the nitrocellulose membrane striped with Anti-NS1 Antibody. For example, in an immunoassay test strip for the detection of anti-Zika antibodies, the control zone contains anti-NS1 antibody immobilized onto the nitrocellulose membrane and the visualization of colloidal gold labeled Zika NS1 antigen is used to confirm component elution, reagent activity, and adequate device performance.

Excess reagent migrates past the Control (C) Line into the absorbent pad [107], which acts as a reservoir for the excess device fluid. An adsorbent pad may be present downstream of the control line in a lateral flow assay test device. Fluid from the membrane (containing unbound complexes and unreacted reagents) moves to the absorbent pad. The absorbent pad serves not only as the end reservoir for device fluid but draws the fluid across the lateral flow assay test strip. Absorbent pads should have sufficient wicking characteristics to prevent backflow of liquid upstream on the assay strip. The absorbent pad may be made of materials known in the art.

A lateral flow assay strip of the invention for the detection of anti-Zika IgM is configured to include a sample receiving area, a blocker area downstream of the sample receiving area, optionally containing Dengue NS1 polypeptides, a conjugate area downstream of the blocker area containing colloidal gold labeled Zika NS1 polypeptides, a first capture zone downstream of the conjugate area containing immobilized protein G, a second capture zone downstream of the first capture zone containing immobilized anti-IgM antibody, and optionally a control line downstream of the second capture zone.

Assay test strips according to the invention may be prepared and assembled using techniques known in the art. Assay test strips according to the invention may be in a housing An assay device according to the invention, comprises a housing having a sample port for applying a liquid biological sample to be tested, and an opening or window to view one or more capture zones and control zones. For example the housing may have an opening to view just the second capture zone and control zone.

An assay method and device according to the invention may further use a developer solution. A developer solution facilitates the capillary flow of the liquid biological sample into the device and onto the assay strip. A developer solution is typically an aqueous solution of surfactants, salts, preservatives, buffering agents, etc. as known in the art. The amount of developer solution used should be sufficient to transport the sample but not so much as to swamp the assay or dilute the results so they cannot be determined.

The invention also provides for assay kits including one or more of the assay devices described herein and methods of use of the kits. The kits may optionally contain any of the buffers, reagents, detection reagents, and so forth that are useful for the practice of the methods of this invention. An assay kit according to the invention comprises an assay device according to the invention and a premeasured vial of developer solution for use with the device.

In a preferred assay for Zika infection according to the invention, the assay is initiated by adding a biological liquid sample directly to the sample port in the neck of a device as shown in FIG. 2 and inserting the collection pad of the device into a developer solution. The developer solution and liquid biological sample are transported up the device via chromatographic lateral flow, hydrating blocker and conjugate pad components, and proceeding through the capture zones, and control line to the absorbent pad.

It is understood that the examples and embodiments described herein are for illustrative purposes only and that various modifications or changes in light thereof will be suggested to persons skilled in the art and are to be included within the spirit and purview of this application and scope of the appended claims.The invention is defined by the claims.

### Examples

### Example 1

Lateral flow assay methods were conducted with monoclonal antibodies with affinity specifically to human IgM were striped on the Test Line, (second capture zone, [105] in FIG. 1) of the lateral flow assay strip. An IgG capture zone ([104] in FIG. 1) was striped with protein G upstream of the test line. A panel of samples collected from patients with Zika infection were tested for the presence of anti-Zika NS1 IgM antibodies. The presence or absence of human anti-Zika NS1 IgM antibodies was verified on ZIKA Detect^{™} Capture ELISA by InBios. As shown in the Table 1 below, human anti-Zika NS1 IgM antibodies were detected on the test line of Zika devices during acute infection but not in samples collected over 90 days post onset of symptom, indicating that IgM but not IgG antibodies were specifically captured on the test line. In the serial draw 1043-TDS-0257, the anti-Zika NS1 IgM antibodies were not detectable at day 3, detectable between 19-62 days but diminished at 91 days post onset of symptom. In the serial draw 1043-TDS-0343, the anti-Zika NS1 IgM antibodies were detectable between 18 and 60 days post onset of symptom. Samples ZIK170713C and ZIK17018B were collected after one year post infection and there were no detectable level of anti-Zika NS1 IgM in these two samples.

**Table 1**

| **Sample ID** | **Days post onset of symptom** | **Test Line** | **Reactivity on InBios Zika IgM ELISA** |
|---|---|---|---|
| 1043-TDS-0257 | 4 | NR | NR |
| 1043-TDS-0257V2 | 19 | R | R |
| 1043-TDS-0257V3 | 26 | R | R |
| 1043-TDS-0257V4 | 35 | R | R |
| 1043-TDS-0257V5 | 40 | R | R |
| 1043-TDS-0257V6 | 47 | R | R |
| 1043-TDS-0257V7 | 54 | R | R |
| 1043-TDS-0257V8 | 62 | R | R |
| 1043-TDS-0257V9 | 91 | NR | N/A |
| 1043-TDS-0343 | 3 | NR | NR |
| 1043-TDS-0343V2 | 18 | R | R |
| 1043-TDS-0343V3 | 25 | R | R |
| 1043-TDS-0343V4 | 32 | R | R |
| 1043-TDS-0343V5 | 38 | R | R |
| 1043-TDS-0343V6 | 46 | R | R |
| 1043-TDS-0343V7 | 53 | R | R |
| 1043-TDS-0343V8 | 60 | R | R |
| ZIK170713C | 369 | NR | NR |
| ZIK170718C | 377 | NR | NR |

| | | | |
|---|---|---|---|
| R: reactive; NR: non-reactive. | | | |

### Example 2

Lateral flow assay methods were conducted with monoclonal antibodies with affinity specifically to human IgM were striped on the Test Line, (second capture zone, [105] in FIG. 1) of the lateral flow assay strip. An IgG capture zone ([104] in FIG. 1) was striped with protein G upstream of the test line. Table 2 shows results of assays in which 0.5 µg Dengue NS1 proteins were present on the blocker pad of the lateral flow devices. Dengue NS1 in the blocker pad reduced the cross-reactivity observed with Dengue positive antibody samples.

**Table 2**

| Sample ID | Without Dengue NS1 | Dengue NS1 added to blocker pad |
|---|---|---|
| 1043-DNG-0209 | R | R |
| 1043-DNG-0212 | R | R |
| 1043-DNG-0235 | R | R |
| 1043-DNG-0256 | R | NR |
| 1043-DNG-0274 | R | R |
| 1043-DNG-0294 | R | NR |
| 0845-0074-02 | R | NR |
| 0845-0074-06 | R | R |
| 0845-0074-09 | R | NR |

| | | |
|---|---|---|
| R: reactive; NR: non-reactive. | | |

Table 3 shows results of assays in which Dengue NS1 proteins were added to the developer solution instead of the blocker pad. To achieve the same reduction of cross-reactivity to Dengue samples, higher amounts of Dengue NS1, 167 µg, compared to 0.5 µg, was needed.

**Table 3**

| Sample ID | Without Dengue NS1 | Dengue NS1 added to developer solution | Dengue NS1 added to blocker pad |
|---|---|---|---|
| DLS16-67408 | R | NR | NR |
| DLS16-67603 | R | NR | NR |
| 1043-DNG-0264 | R | R | R |

| | | | |
|---|---|---|---|
| R: reactive; NR: non-reactive. | | | |

Table 4 shows results of assays in which Dengue NS1 proteins were immobilized on the lateral flow assay strip either upstream or downstream of the protein G line, but upstream of of the IgM capture line. The reduction of cross-reactivity was not significant in these configurations.

**Table 4**

| Sample ID | Without Dengue NS1 | Dengue NS1 added to blocker pad | Dengue NS1 line upstream of IgG capture zone | Dengue NS1 line between capture zones |
|---|---|---|---|---|
| DLS 1534105 | R | NR | R | R |
| DLS 1667586 | R | NR | R | R |

| | | | | |
|---|---|---|---|---|
| R: reactive; NR: non-reactive. | | | | |

## Claims

1. A lateral flow assay strip for the detection of an antibody isotype associated with an acute immune response to a Zika virus infection, comprising:
a) a sample receiving area for a biological sample;
b) a blocker area located downstream of the sample receiving area, wherein the blocker area comprises Dengue NS1 polypeptides;
c) a conjugate area on the lateral flow assay test strip located downstream from the blocker area, wherein the conjugate area comprises labeled antigens that specifically bind to Zika NS1-specific antibodies in the biological sample, wherein the labeled antigens are labeled Zika NS1 polypeptides;
d) a first capture zone on the lateral flow assay test strip located downstream from the conjugate area, wherein the first capture zone comprises capture reagents that specifically capture IgG isotype antibodies;
e) a second capture zone on the lateral flow assay test strip located downstream from the conjugate area, wherein the second capture zone comprises capture reagents that specifically capture IgM isotype antibodies.

2. The lateral flow assay strip according to claim 1, wherein the label of the labeled Zika NS1 polypeptides is colloidal gold.

3. The lateral flow assay strip according to any one of claims 1 or 2, wherein the capture reagent in the first capture zone is protein G.

4. The lateral flow assay strip of any one of claims 1-3, wherein the capture agent in the second capture zone is an anti-human IgM antibody, or fragment thereof.

5. Use of the lateral flow assay strip of any one of claims 1-4 for detection of an acute immune response against Zika virus.

## Patentansprüche

1. Teststreifen für lateralen Fluss zur Detektion eines Antikörper-Isotyps, der mit einer akuten Immunantwort gegen eine Zika-Virus-Infektion assoziiert ist, umfassend:
a) einen Probeaufnahmebereich für eine biologische Probe;
b) einen Blockierungsbereich, der stromabwärts zu dem Probeaufnahmebereich lokalisiert ist, wobei der Blockierungsbereich Dengue-NS1-Polypeptide umfasst;
c) einen Konjugatbereich auf dem Teststreifen für lateralen Fluss, der stromabwärts zu dem Blockierungsbereich lokalisiert ist, wobei der Konjugatbereich markierte Antigene umfasst, die spezifisch an Zika-NS1-spezifische Antikörper in der biologischen Probe binden, wobei die markierten Antigene markierte Zika-NS1-Polypeptide sind;
d) eine erste Erfassungszone auf dem Teststreifen für lateralen Fluss, die stromabwärts zu dem Konjugatbereich lokalisiert ist, wobei die erste Erfassungszone Erfassungsreagenzien umfasst, die spezifisch IgG-Isotyp-Antkörper erfassen;
e) eine zweite Erfassungszone auf dem Teststreifen für lateralen Fluss, die stromabwärts zu dem Konjugatbereich lokalisiert ist, wobei die zweite Erfassungszone Erfassungsreagenzien umfasst, die spezifisch IgM-Isotyp-Antkörper erfassen.

2. Teststreifen für lateralen Fluss gemäß Anspruch 1, wobei die Markierung des markierten Zika-NS1-Polypeptids kolloidales Gold ist.

3. Teststreifen für lateralen Fluss gemäß einem beliebigen der Ansprüche 1 oder 2, wobei das Erfassungsreagenz in der ersten Erfassungszone Protein G ist.

4. Teststreifen für lateralen Fluss nach einem beliebigen der Ansprüche 1-3, wobei das Erfassungsreagenz in der zweiten Erfassungszone ein anti-humaner IgM-Antikörper oder ein Fragment davon ist.

5. Verwendung des Teststreifens für lateralen Fluss nach einem beliebigen der Ansprüche 1-4 zur Detektion einer akuten Immunantwort gegen Zika-Virus.

## Revendications

1. Bandelette d'essai à flux latéral pour la détection d'un isotype d'anticorps associé à une réponse immunitaire aiguë à une infection par le virus Zika, comprenant:
a) une zone de réception d'échantillon pour un échantillon biologique;
b) une zone de blocage située en aval de la zone de réception de l'échantillon, dans laquelle la zone de blocage comprend des polypeptides NS1 de la dengue;
c) une zone conjuguée sur la bandelette d'essai à flux latéral située en aval de la zone de blocage, dans laquelle la zone conjuguée comprend des antigènes marqués qui se lient spécifiquement aux anticorps spécifiques du Zika NS1 dans l'échantillon biologique, les antigènes marqués étant des polypeptides marqués du Zika NS1 ;
d) une première zone de capture sur la bandelette d'essai à flux latéral située en aval de la zone conjuguée, dans laquelle la première zone de capture comprend des réactifs de capture qui capturent spécifiquement les anticorps d'isotype IgG ;
e) une deuxième zone de capture sur la bandelette d'essai à flux latéral située en aval de la zone conjuguée, dans laquelle la deuxième zone de capture comprend des réactifs de capture qui capturent spécifiquement les anticorps d'isotype IgM.

2. Bandelette d'essai à flux latéral selon la revendication 1, dans laquelle l'étiquette des polypeptides Zika NS1 marqués est de l'or colloïdal.

3. Bandelette d'essai à flux latéral selon l'une des revendications 1 ou 2, dans laquelle le réactif de capture dans la première zone de capture est la protéine G.

4. Bandelette d'essai à flux latéral selon l'une quelconque des revendications 1-3, dans laquelle le réactif de capture dans la deuxième zone de capture est un anticorps IgM anti-humain ou un fragment de celui-ci.

5. Utilisation de la bandelette d'essai à flux latéral selon l'une quelconque des revendications 1-4 pour la détection d'une réponse immunitaire aiguë contre le virus Zika.
